# EUROPÄISCHE PATENTSCHRIFT

(11) **EP 0 871 405 B1**
(45) Veröffentlichungstag und Bekanntmachung des Hinweises auf die Patenterteilung: **14.02.2001**
(21) Anmeldenummer: 96942259.1
(22) Anmeldetag: 20.11.1996
(51) Int. Cl.: A61B 18/14

(54) **BIPOLARES HOCHFREQUENZ-CHIRURGIEINSTRUMENT**
BIPOLAR HIGH-FREQUENCY SURGICAL INSTRUMENT
INSTRUMENT CHIRURGICAL BIPOLAIRE A HAUTE FREQUENCE

(30) Priorität: 20.11.1995 DE 19543258
(43) Veröffentlichungstag der Anmeldung: 21.10.1998
(73) Patentinhaber: Storz Endoskop GmbH, 8200 Schaffhausen (CH)
(72) Erfinder: NOVAK, Pavel, CH-8207 Schaffhausen (CH); WATTIEZ, Arnaud, Centre de Chirurgie Endoscopique, F-63000 Clermont-Ferrand (FR)
(74) Vertreter: Münich, Wilhelm, Dr.
(86) Internationale Anmeldenummer: DE9602214
(87) Internationale Veröffentlichungsnummer: WO9718766

(56) Entgegenhaltungen:
- EP-A- 0 400 288
- EP-A- 0 455 321
- EP-A- 0 536 440
- WO-A-91/17717
- WO-A-94/20025
- FR-A- 2 700 685
- US-A- 5 401 272

## Beschreibung

### Technisches Gebiet

Die Erfindung bezieht sich auf ein Hochfrequenz-Chirurgieinstrument gemäß den Oberbegriffen des Patentanspruches 1.

Derartige Hochfrequenz-Chirurgieinstrumente werden insbesondere als bipolare Instrumente eingesetzt.

### Stand der Technik

Es ist bekannt, mit hochfrequenten Strömen, deren Frequenz beispielsweise im Bereich zwischen 500 kHz und 1 MHz liegt, Gewebe zu schneiden oder zu koagulieren. Dabei unterscheidet man grundsätzlich zwischen monopolarer und bipolarer Betriebsart:

Bei der monopolaren Betriebsart fließt der Strom zwischen einer großflächigen Neutralelektrode und einer kleinen Arbeitselektrode, die auch als Aktivelektrode bezeichnet wird. Die Aktivelektrode kann insbesondere die Form einer Spitze oder einer kleinen Kugel haben. Aufgrund der hohen Stromdichte an der Aktivelektrodenspitze kommt es nur in der Umgebung dieser Spitze zum Austrocknen oder Verbrennen des Gewebes, wobei entsprechend der Stromform (Sinus, Sinus mit Unterbrechungen bzw. Impulsform) eine Schnittwirkung oder verstärkt eine Koagulation auftritt.

Bei der bipolaren Betriebsart erfolgt der Stromfluß zwischen zwei kleinen Elektroden, die ein Stück Gewebe einschließen. In der Vergangenheit ist diese Betriebsart im wesentlichen nur zum Koagulieren und in der Praxis nur in geringem Umfang zum Schneiden benutzt worden.

Im Gegensatz zur bipolaren Betriebsart fließt bei der monopolaren Betriebsart der Strom durch große Bereiche des menschlichen Körpers. Damit besteht die Gefahr, daß - vor allem bei einer nicht 100 % sachgemäßen Anwendung - nicht nur das Gewebe in unmittelbarer Nähe der Aktivelektrode durch den Stromfluß in der gewünschten Weise beeinflußt wird, sondern daß beispielsweise durch Stromfluß im Bereich der Herzgegend eine Störung der Herzfunktion auftreten kann. Darüber hinaus kann eine koagulierende Wirkung auch bei Gewebesträngen etc. auftreten, die sich weit entfernt von dem eigentlich zu koagulierenden Gebiet befinden.

Aus diesem Grunde wird seit einiger Zeit versucht, eine Schnittwirkung auch bei der bipolaren Betriebsart zu erzielen.

So ist es beispielsweise bekannt, eine "Triode" zu verwenden, bei der durch die Formgebung der beiden Elektroden, von denen die eine eine Spitze und die andere eine Halbkugel ist, die Verhältnisse bei der monopolaren Betriebsart nachbildet werden. Aufgrund dieser Form kommt eine Schnittwirkung nur an der Spitze der Elektroden zustande, so daß sehr präzise geschnitten werden kann. Diese Form der Elektroden erlaubt es jedoch nicht, das zu schneidende Gewebe zu greifen.

Eine bipolare Koagulationszange ist aus dem deutschen Gebrauchsmuster 92 15 590 bekannt. Diese Koagulationszange weist zwei Elektroden auf, die als Backen einer Zange ausgebildet sind und damit als Klemmelektroden dienen können, zwischen denen das zu schneidende Gewebe gehalten bzw. eingeklemmt wird. Durch Anlegen eines HF-Stroms eines HF-Generators kann eine Koagulation des Gewebes ausgeführt werden. Diese bekannte bipolare Koagulationszange eignet sich ausschließlich zum Koagulieren, nicht jedoch zum Schneiden von Gewebe. Darüberhinaus weist diese Koagulationszange keinen Kanal auf, der als Spül- und/oder Absaugkanal dienen könnte, durch den beispielsweise Gewebestücke abgesaugt werden könnten.

Entsprechendes gilt für die aus den US-PS 5 269 782, 5 282 799, 5 258 006 und 5 290 285 sowie der EP-A-0 596 436 bekannten bipolaren Zangen bzw. Instrumente.

Ein weiteres bipolares Koagulations-Instrument ist aus der EP 0 536 440 bekannt. Dieses Instrument für die Hochfrequenzchirurgie eignet sich zwar zum wahlweisen Schneiden oder Koagulieren, es ist jedoch nicht für beide Betriebsarten optimal ausgelegt.
Die in den vorstehend genannten Druckschriften - auf die im Übrigen zur Erläuterung aller hier nicht im einzelnen beschriebenen Einzelheiten ausdrücklich bezug genommen wird - beschriebenen bipolaren Anordnungen haben damit den Nachteil, daß sie keinen Spül- und/oder Absaugkanal aufweisen. Darüberhinaus können diese bipolaren Elektroden nicht mit herkömmlichen Hochfrequenz-Chirurgieinstrumenten, wie sie bei vielen Ärzten bzw. Kliniken bereits im Rahmen vorhandener Endoskopie-Systeme vorhanden sind, verwendet werden. Weiterhin sind sie entweder nur zum Koagulieren und nicht zum Schneiden geeignet, oder eine der beiden Funktionen - meist die Schneid-Funktion - ist nicht optimal ausführbar.

Die EP 0 400 288 offenbart ein Hochfrequenz-Chirurgieinstrument zur bipolaren Koagulation gemäß dem Oberbegriff des Patentanspruches 1. Dieses Instrument weist einen Spülkanal auf. Damit stellt diese Lösung einen der Ausgangspunkte zu den Überlegungen für die weitere Verbesserung des bekannten Standes der Technik dar.

Die EP 0 455 321 offenbart einen feststehenden Abstandhalter zwischen den Schneidelektroden.

### Darstellung der Erfindung

Der Erfindung liegt die Aufgabe zugrunde, ein insbesondere für bipolare Anwendungen geeignetes Hochfrequenz-Chirurgieinstrument mit zwei Elektroden anzugeben, bei dem wenigstens ein Kanal, der als Spül- und/oder Absaugkanal dienen kann, integriert ist, und das sowohl für die Betriebsart "koagulieren" als auch "schneiden" optimal geeignet ist. Es besteht insbesondere die Aufgabe, die Schnittwirkung ausgehend von den beschriebenen Nachteilen bekannter Lösungen zu optimieren.

Eine erfindungsgemäße Lösung dieser Aufgabe ist im Patentanspruch 1 gegeben.

Bei der im Anspruch 1 angegebenen Ausführungsform ist ein elektrisch nicht leitender Abstandhalter vorgesehen, der beim Schneiden von Gewebe zwischen die Elektroden, d.h. zwischen die Backen der Zange eingebracht wird. Weiterhin ist wenigstens ein Kanal vorgesehen, der als Spül- und/oder Absaugkanal dient.
Durch den erfindungsgemäß vorgesehenen elektrisch nicht leitenden Abstandhalter haben die beiden Backen der Zange beim Schneiden einen Mindestabstand und damit gleichzeitig (in der Regel) einen genau definierten Abstand, der typischerweise 1 mm (Anspruch 10) betragen kann, so daß bei einer Beaufschlagung mit einem HF-Strom mit geeigneter - aus dem Stand der Technik bekannter - Wellenform eine optimale Schneidwirkung erhalten wird.

Der vorgesehene Kanal kann dadurch gebildet werden, daß die Halter der Backen wenigstens einen Kanal einschließen, der als Spül- und/oder Absaugkanal (Anspruch 11) dienen kann. Alternativ kann der Kanal auch dadurch gebildet werden, daß in an sich bekannter Weise, die Backen der Zange in einem Außenrohr verlaufen (Anspruch 12), das den Spül-und/oder Absaugkanal einschließt (Anspruch 13). Selbstverständlich können auch der Spülkanal und der Absaugkanal getrennt sein: So ist es möglich, daß einer der beiden Kanäle von den Haltern der Backen eingeschlossen wird, und der andere Kanal ein konzentrischer Kanal ist, der von dem Außenrohr begrenzt wird.

Bei der im Anspruch 2 angegebenen Weiterbildung enden die als Elektroden dienenden Backen der Zangen in einer Spitze. Hierdurch wird nicht nur das Greifen und Festhalten des Gewebes erleichtert, sondern es ist auch ein definierter Stromfluß sichergestellt, der bevorzugt von den Spitzen ausgeht.

Besonders bevorzugt ist es dabei, wenn gemäß Anspruch 3 die Backen vor der Spitze eine kleinere Breite als am hinteren Bereich haben.

Die Spitze kann dabei in Richtung der Längsachse des Instruments angeordnet sein. Besonders bevorzugt ist es jedoch, wenn die Spitze einen Winkel mit der Längsachse des Instruments einschließt, der beispielsweise 30°, bevorzugt 90°, 120° oder wiederum bevorzugt 180° betragen kann. Dabei können die Backen austauschbar sein, so daß in ein Instrument Backen mit unterschiedlichen Winkeln zwischen der Spitze und der Längsachse des Instruments einsetzbar sind. Hierdurch kann ein "Grund-Instrument" optimal an die jeweiligen Operationsbedingungen angepaßt werden.

Eine um 90° abgewinkelte Spitze hat den Vorteil, daß sie in einfacher Weise das Greifen von Gewebe erlaubt.

Im Falle einer um 180° gebogenen Spitze (Anspruch 7) ist es weiterhin bevorzugt, wenn die Spitzen innerhalb der Backenkontur liegen. Hierdurch ist es einerseits möglich, das Gewebe sicher zu greifen und andererseits einen definierten Stromfluß, der zum Schneiden besonders geeignet ist, sicherzustellen.

Der erfindungsgemäß vorgesehene Abstandshalter ist gemäß Anspruch 8 bevorzugt ein Schieber, der zum Schneiden des Gewebes derart betätigt wird, daßer nach vorne zwischen die Backen geschoben wird, so daß diese einen Mindestabstand haben. Der als Schieber ausgebildete Abstandshalter kann weiterhin gemäß Anspruch 9 eine Schneide insbesondere an seiner Vorderkante aufweisen, so daß er auch zum mechanischen Schneiden von Gewebe eingesetzt werden kann. Hierzu ist er bevorzugt so ausgebildet, daß er über das distale Ende der Elektroden bzw, Backen vorgeschoben werden kann.

Das erfindungsgemäße Instrument kann einen Grundaufbau ähnlich bekannten Instrumenten haben, so daß es mit vorhandenen Instrumenten aus einem Endoskopie-System, wie beispielsweise vorhandenen Trokarschäften oder vorhandenen Endoskopen verwendet werden kann.

Insbesondere können die Backen aus einem aufgebogenen elastischen Material bestehen, so daß sie im "Normalzustand" geöffnet sind, und erst durch eine Relativverschiebung zwischen Backen und Äußenrohr geschlossen werden, wie dies beispielsweise in der EP-A 0 598 453 beschrieben ist. Dabei ist es bevorzugt, wenn - wie ebenfalls in dieser Druckschrift beschrieben - zum Schließen der Backen nicht diese zurückgezogen werden, sondern das Außenrohr nach vorne verschoben wird (Anspruch 15), da dann zum Schließen der Backen keine Ortsveränderung dieser erforderlich ist, die unter Umständen dazu führen könnte, daß nicht der gewünschte Gewebeabschnitt koaguliert bzw. geschnitten wird.

Bei dieser Ausgestaltung der Zange ist es weiterhin bevorzugt, wenn die Bakken aus einem superelastischen Material und insbesondere einem shape-memory-Material bestehen (Ansprüche 16 und 17).
Alternativ kann die erfindungsgemäß eingesetzte Zange auch derart ausgebildet sein, daß die Backen an wenigstens einem Gelenk angelenkt sind, und über (wenigstens) eine Schub- oder Zugstange betätigt werden (Anspruch 18).

In jedem Falle ist es von Vorteil, wenn gemäß Anspruch 19 die Halter der Backen auch als Zuleitungen für die als Elektroden dienenden Backen dienen und gegenüber der Umgebung elektrisch isoliert sind.

Das erfindungsgemäße Instrument kann - beispielsweise in einen Trokarschaft eingesetzt - zur bipolaren Behandlung und insbesondere zur Koagulation oder zum Schneiden von Gewebe bei endoskopischen Eingriffen - aber nicht nur bei derartigen Eingriffen - dienen. Darüberhinaus eignet sich das Instrument auch zur monopolaren Behandlung. Hierzu ist es bevorzugt, wenn gemäß Anspruch 20 ein kombinierter Anschluß an den monopolaren und den bipolaren Ausgang eines (bekannten) Hochfrequenzgenerators vorgesehen ist.

In jedem Falle ist das erfindungsgemäße Instrument besonders als multifunktionales Instrument zum bipolaren Schneiden, monopolaren oder bipolaren Koagulieren, zum Greifen oder Spülen und gegebenenfalls zum Schneiden mittels der Schneide des Schiebers geeignet (Anspruch 21).
Hierzu kann das Instrument aus einem an sich bekannten Zangenteil, wie es bei endoskopischen Operations-Instrumenten bekannt ist, und einem an sich bekannten Multifunktionshandgriff bestehen, mit dem die einzelnen Funktionen gesteuert werden. Entsprechende Zangenteile und Multifunktionshandgriffe werden beispielsweise von der Karl Storz GmbH & Co., Tuttlingen, Deutschland hergestellt.

Ein Hochfrequenz-Chirurgieinstrument, wie es aus der EP-A 0 536 440 bekannt ist, kann derart weitergebildet, werden daß der als Rohr ausgebildete Halter der ringförmigen Elektrode und der als Stab ausgebildete Halter der spitzenförmigen Elektroden einen Kanal einschließen, der als Spülund/oder Absaugkanal dient. Zur Optimierung der Betriebsart "Schneiden" ist der als Stab ausgebildete Halter der spitzenförmigen Elektrode parallel zur Längsachse des Instruments verschiebbar, so daß immer der optimale Abstand zwischen der ringförmigen Elektrode und der Spitzenelektrode eingestellt werden kann.

Darüberhinaus ist es aufgrund dieser nicht zur Erfindung gehörenden Ausbildung möglich, die Spitze hinter die ringförmige Elektrode zurückziehen, so daß beim Einführen des Instruments die Verletzungsgefahr minimiert wird.

Diese Ausführungsform des nicht zur Erfindung gehörenden Instruments kann ebenfalls einen kombinierten Anschluß an den monopolaren und den bipolaren Ausgang eines Hochfrequenzgenerators aufweisen.

Auch bei dieser Ausführungsform können die Elektrodenhalter als Zuleitungen für die Elektroden dienen. Hierzu sind sie gegenüber der Umgebung elektrisch isoliert.

Im einer weiteren nicht zur Erfindung gehörenden Ausführungsform ist angegeben, daß die ringförmige Elektrode in Normalstellung in Richtung der Längsachse des Instruments hinter der spitzenförmigen Elektrode angeordnet ist, wobei der Abstand der ringförmigen Elektrode von der spitzenförmigen Elektrode in Richtung der Längsachse kleiner oder höchstens gleich dem Durchmesser der ringförmigen Elektrode ist.

### Kurze Beschreibung der Zeichnungen

Die Erfindung wird nachstehend anhand von Ausführungsbeispielen unter Bezugnahme auf die Zeichnung näher beschrieben, in der zeigen.
Fig. 1 ein nicht zur Erfindung gehözendes Ausführungsbeispiel, und
Fig. 2a und b zwei Alternativen eines Ausführungsbeispiels der Erfindung.

### Darstellung von Ausführungsbeispielen

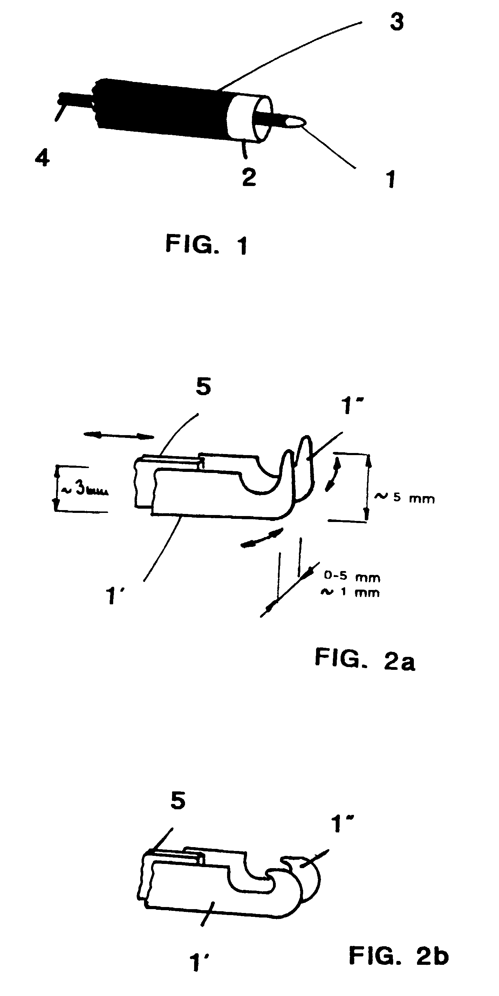

Fig. 1 zeigt den distalen Endbereich eines ansonsten nicht dargestellten bipolaren Hochfrequenz-Chirurgieinstruments mit zwei Elektroden 1 und 2. Das HF-Chirurgieinstrument kann in an sich bekannter Weise ausgebildet sein, so daß an dieser Stelle auf eine ausführliche Beschreibung verzichtet werden kann. Insbesondere kann es sich bei dem Instrument es sich um ein bekanntes HF-Instrument handeln, wie es beispielsweise von der Karl Storz GmbH & Co. serienmäßig hergestellt wird.

Die Elektrode 1 hat die Form einer Spitze und dient als Schneidelektrode. Die Elektrode 2 hat die Form eines Rings. Die Elektrode 1 wird von einem als Stab ausgebildeten Halter 4 gehalten, während die Elektrode 2 von einem Rohr 3 gehalten wird. Der Stab 4 und das Rohr 3 schließen einen Kanal ein, der als Spül- und/oder Absaugkanal dienen kann.

Die Elektroden-Halter 3 und 4 dienen auch als Zuleitungen für die Elektroden von dem proximal angeordneten Anschluá für einen nicht dargestellten HF-Generator und sind gegenüber der Umgebung elektrisch isoliert.

Die ringförmige Elektrode 2 ist dabei in Richtung der Längsachse des Instruments hinter der spitzenförmigen Elektrode 1 angeordnet, wobei bevorzugt der Abstand der ringförmigen Elektrode 2 von der spitzenförmigen Elektrode 1 in Richtung der Längsachse kleiner oder höchstens gleich dem Durchmesser der ringförmigen Elektrode 2 ist.

Bei einem Ausführungsbeispiel ist die spitzenförmige Elektrode 1 in Richtung der Längsachse des Instruments, also in Richtung des Stabs 4 gegenüber der ringförmigen Elektrode 2 verschiebbar und insbesondere "hinter die distale "Endfläche" der Elektrode 2 zurückziehbar.

Das Vorsehen eines Kanals, der eine Spül- und/oder Absaugmöglichkeit gibt, hat neben ergonomischen Vorteilen durch die Vereinigung von HF-Schneide- und Spülinstrument auch noch den Vorteil, daß durch gleichzeitiges Spülen während des Schneidens die Gewebeschädigung minimiert wird, und abgeschnittene Teilchen schnell aus dem Körper entfernt werden können.

Die in Fig. 1 dargestellte nicht zur Erfindung gehörende bipolare Elektrode ist bevorzugt zum Schneiden ausgelegt.

Zwei Ausführungsformen der Erfindung, die gleichrangig zum Schneiden und bipolaren Koagulieren verwendbar sind, sind in Fig. 2 dargestellt.

Das in Fig. 2 dargestellte Hochfrequenz-Chirurgieinstrument hat die Form einer Zange, wobei die Elektroden 1' und 1" als Backen dieser Zange ausgebildet sind. Die eigentliche Zange kann beispielsweise ähnlich bekannten bipolaren HF-Zangen der Karl Storz GmbH & Co, Tuttlingen, Deutschland, oder in einer Weise ausgebildet sein, wie sie beispielsweise in der EP 0 589 453 A2 beschrieben ist. Selbstverständlich ist aber auch jeder andere bekannte HF-Zangen-Aufbau einsetzbar.

Bei beiden in Fig. 2 dargestellten Ausführungsbeispielen wird bevorzugt eine Zange verwendet, bei der die Backen 1' und 1" aus einem aufgebogenem elastischen Material bestehen. Ferner weist die Zange ein ebenfalls nicht dargestelltes Außenrohr auf, das die Halter der Backen 1' und 1" sowie weitere, nachstehend noch näher erläuterte Teile der Zange aufnimmt. Durch eine Relativverschiebung zwischen Backen 1' und 1" und Außenrohr werden die Backen in das Außenrohr eingezogen und damit geschlossen. Dabei ist es bevorzugt, wenn zum Schließen der Backen das Außenrohr nach vorne verschoben wird, da es bei dieser Ausgestaltung nicht erforderlich ist, die Zangen-Backen, die das Gewebe greifen, zum Schließen der Backen zu verschieben. Insbesondere können die Backen aus einem superelastischen Material, wie einem Shape-Memory-Material bestehen.

Weiterhin ist es bevorzugt, wenn die Halter der Backen wenigstens einen Kanal einschließen, der als Spül- und/ oder Absaugkanal dient. Gegebenenfalls kann ein weiterer Kanal vorgesehen sein, der zwischen den Haltern der Bakken und dem nicht dargestellten Außenrohr vorgesehen ist.

Mit der erfindungsgemäßen Zange kann damit ein Eingriff beispielsweise wie folgt vorgenommen werden:

Zunächst wird beispielsweise mit einem Trokar mit eingesetztem Trokardorn ein Zugang zu der zu behandelnden Körperhöhle geschaffen. Alternativ kann auch ein "natürlicher" Zugang benutzt werden.
Anschließend wird durch den Trokar (auch als Trokarschaft bezeichnet) - gegebenenfalls unter Beobachtung mit einem Endoskop - das erfindungsgemäße Instrument in die Körperhöhle eingesetzt. Der Gewebeabschnitt, der koaguliert oder abgeschnitten werden soll, wird mit den als Zangen-Backen ausgebildeten Elektroden 1' und 1" gegriffen. Durch "Vorschieben" des Außenrohrs werden die Backen geschlossen und das Gewebe fixiert. Nunmehr werden die beiden Elektroden mit HF-Strom eines nicht dargestellten Hochfrequenz-Generators beaufschlagt, so daß das Gewebe geschnitten oder - je nach zeitlichem Stromverlauf - koaguliert wird.

Abgeschnittene und nicht fixierte Gewebeteile können durch den erfindungsgemäß vorgesehenen Kanal abgesaugt werden.

Bei beiden, in den Fig. 2a und 2b dargestellten Ausführungsbeispielen enden beide Elektroden in einer Spitze, die mit der Längsachse des Instruments einen Winkel einschließt. Bei dem in Fig. 2a dargestellten Beispiel beträgt der Winkel 90°, während er bei dem in Fig. 2b gezeigten Beispiel 180° beträgt. Damit ist - insbesondere dann, wenn die Backen austauschbar sind, so daß Backen mit unterschiedlichen Winkeln der Spitze einsetzbar sind. eine optimale Anpassung an den jeweiligen Behandlungsfall möglich. Ausdrücklich wird auf die in Fig. 2a angegebenen Maße hingewiesen, die bevorzugt für die erfindungsgemäße Ausbildung sind.

Da der Abstand zwischen den beiden Elektroden dann, wenn eine Schneidwirkung erzielt werden soll, kritisch ist, ist durch den zwischen den Elektroden vorgesehenen Kanal ein elektrisch nicht leitender Abstandshalter 5 einbringbar, der den Abstand zwischen den Elektroden 1' und 1" beim Schneiden auf ca. 1 mm einstellt. Soll koaguliert werden, wird der Abstandshalter entfernt, so daß der Abstand zwischen den Elektroden typischerweise zwischen nahezu 0 und 5 mm eingestellt werden kann.

Um die Schneidwirkung zu verbessern, sind beide Elektroden so ausgebildet, daß sie vor der Spitze eine kleinere Breite als im hinteren Bereich haben. Hierdurch wird der Stromfluß vorteilhaft beeinflußt.

Die nicht näher dargestellten Halter der Backen 1' und 1" dienen auch als Zuleitungen für die Backen und sind gegenüber der Umgebung elektrisch isoliert, so daß sie den HF-Strom von dem nicht dargestellten HF-Anschluß zum distalen Ende leiten können.

Alternativ können die Backen an wenigstens einem Gelenk angelenkt sein und über eine Schub- oder Zugstange betätigt werden.

Weiterhin kann der Schieber 5 an seinem distalen Ende eine Schneide zum Abschneiden von Gewebe aufweisen. Das Instrument kann ferner einen kombinierten Anschluß an den monopolaren und den bipolaren Ausgang des Hochfrequenz-Generators aufweisen.

## Patentansprüche

1. Hochfrequenz-Chirurgieinstrument mit zwei Elektroden (1',1"), die als Backen einer Zange ausgebildet sind, an die ein HF-Strom eines HF-Generators derart anlegbar ist, daß ein hochfrequenter Strom zwischen den Backen durch den zu behandelnden Gewebebereich fließt und daß wenigstens ein Kanal vorgesehen ist, der als Spül- und/oder Absaugkanal dient,
**dadurch gekennzeichnet,** daß zum wahlweisen Schneiden und Koagulieren ein elektrisch nicht leitender Abstandshalter (5) vorgesehen ist, der beim Schneiden von Gewebe zwischen die Elektroden eingebracht wird, so daß die beiden Backen der Zange beim Schneiden einen Mindestabstand haben.

2. Instrument nach Anspruch 1,
**dadurch gekennzeichnet,** daß die als Elektroden dienenden Backen (1',1") der Zange in einer Spitze enden.

3. Instrument nach Anspruch 2,
**dadurch gekennzeichnet,** daß die Backen (1',1") vor der Spitze eine kleinere Breite als in ihrem hinteren Bereich haben.

4. Instrument nach Anspruch 2 oder 3,
**dadurch gekennzeichnet,** daß die Spitze einen Winkel mit der Längsachse des Instruments einschließt.

5. Instrument nach Anspruch 4,
**dadurch gekennzeichnet,** daß die Backen (1',1") austauschbar sind, so daß Bakken mit unterschiedlichen Winkeln der Spitze einsetzbar sind.

6. Instrument nach Anspruch 4 oder 5,
**dadurch gekennzeichnet,** daß der Winkel 0°, 30°, 90°, 120° oder 180° beträgt.

7. Instrument nach Anspruch 6,
**dadurch gekennzeichnet,** daß im Falle einer um 180° gebogenen Spitze die Spitze innerhalb der Backenkontur liegt.

8. Instrument nach einem der Ansprüche 1 bis 7,
**dadurch gekennzeichnet,** daß der Abstandhalter (5) ein Schieber ist.

9. Instrument nach Anspruch 8,
**dadurch gekennzeichnet,** daß der Abstandhalter (5) eine Schneide aufweist.

10. Instrument nach einem der Ansprüche 1 bis 9,
**dadurch gekennzeichnet,** daß der Abstand zwischen den Elektroden (1',1") beim Schneiden ca. 1 mm beträgt.

11. Instrument nach einem der Ansprüche 1 bis 10,
**dadurch gekennzeichnet,** daß die Halter der Backen (1',1") wenigstens einen Kanal einschließen, der als Spül- und/oder Absaugkanal dient.

12. Instrument nach einem der Ansprüche 1 bis 11,
**dadurch gekennzeichnet,** daß die Backen (1',1") der Zange in ein Außenrohr eingesetzt sind.

13. Instrument nach Anspruch 12,
**dadurch gekennzeichnet,** daß der als Spül- und/oder Absaugkanal dienende Kanal in dem Außenrohr vorgesehen ist.

14. Instrument nach Anspruch 12 oder 13,
**dadurch gekennzeichnet,** daß die Backen (1',1") aus einem aufgebogenen elastischen Material bestehen und durch eine Relativverschiebung zwischen Bakken und Außenrohr geschlossen werden.

15. Instrument nach Anspruch 14,
**dadurch gekennzeichnet,** daß zum Schließen der Backen (1',1") das Außenrohr nach vorne verschoben wird.

16. Instrument nach Anspruch 14 oder 15,
**dadurch gekennzeichnet,** daß die Backen (1',1") aus einem superelastischen Material bestehen.

17. Instrument nach Anspruch 16,
**dadurch gekennzeichnet,** daß das Material ein "shape-memory-Material" ist.

18. Instrument nach einem der Ansprüche 1 bis 17,
**dadurch gekennzeichnet,** daß die Backen (1',1") an wenigstens einem Gelenk angelenkt sind, und daß die Backen über eine Schub- oder Zugstange betätigt werden.

19. Instrument nach einem der Ansprüche 1 bis 18,
**dadurch gekennzeichnet,** daß die Halter der Backen (1',1") auch als Zuleitungen für die als Elektroden dienenden Backen dienen und gegenüber der Umgebung elektrisch isoliert sind.

20. Instrument nach einem der Ansprüche 1 bis 19,
**dadurch gekennzeichnet,** daß das Instrument einen kombinierten Anschluß an den monopolaren und bipolaren Ausgang des Hochfrequenz-Generators aufweist.

21. Instrument nach einem der Ansprüche 1 bis 20,
**dadurch gekennzeichnet,** daß das Instrument ein multifunktionales Instrument zum bipolaren Schneiden, monopolaren oder bipolaren Koagulieren, zum Greifen, Spülen und gegebenenfalls zum Schneiden mittels der Schneide des Schiebers ist.

22. Instrument nach Anspruch 21,
**dadurch gekennzeichnet,** daß das Instrument einen an sich bekannten Multifunktionshandgriff aufweist, mit dem die einzelnen Funktionen gesteuert werden können.

## Claims

1. High-frequency surgical instrument comprising two electrodes (1', 1") configured as jaws of a pair of forceps, to which an HF current from an HF generator is applicable in such a way that a high-frequency current will flow between the jaws through the tissue region to be treated, and that at least one duct is provided which serves as rinsing/irrigating and/or exhausting duct,
**characterised** in that for optionally severing and coagulating an electrically non-conducting spacer (5) is provided which is introduced between said electrodes when tissue is severed, such that the two forceps jaws will have a minimum spacing during the severing operation.

2. Instrument according to Claim 1,
**characterised** in that said jaws (1', 1") of said pair of forceps, which serve as electrodes, terminate in a tip.

3. Instrument according to Claim 2,
**characterised** in that said jaws (1', 1") have a width ahead of the tip which is smaller than the width in their rear zone.

4. Instrument according to Claim 2 or 3,
**characterised** in that said tip encloses an angle relative to the longitudinal axis of the instrument.

5. Instrument according to Claim 4,
**characterised** in that said jaws (1', 1") are exchangeable such that jaws with different angles of the tip may be inserted.

6. Instrument according to Claim 4 or 5,
**characterised** in that said angle amounts to 0°, 30°, 90°, 120° or 180°.

7. Instrument according to Claim 6,
**characterised** in that in the event of a tip bent by 180° the tip is located within the jaw contour.

8. Instrument according to any of the Claims 1 to 7,
**characterised** in that said spacer (5) is a slide.

9. Instrument according to Claim 8,
**characterised** in that said spacer (5) presents a blade.

10. Instrument according to any of the Claims 1 to 9,
**characterised** in that in the severing operation the spacing between said electrodes (1', 1") amounts to roughly 1 mm.

11. Instrument according to any of the Claims 1 to 10,
**characterised** in that said mounts of said jaws (1', 1") enclose at least one duct serving as rinsing/irrigating and/or exhausting duct.

12. Instrument according to any of the Claims 1 to 11,
**characterised** in that said jaws (1', 1") of the pair of forceps are inserted into an outside tube.

13. Instrument according to Claim 12,
**characterised** in that said duct serving as rinsing/irrigating and/or exhausting duct is provided in said outside tube.

14. Instrument according to Claim 12 or 13,
**characterised** in that said jaws (1', 1") consist of a bent-up resilient material and are closed by a displacement of said jaws relative to said outside tube.

15. Instrument according to Claim 14,
**characterised** in that said for closing said jaws (1', 1") said outside tube is shifted in the forward direction.

16. Instrument according to Claim 14 or 15,
**characterised** in that said jaws (1', 1") consist of a super-elastic material.

17. Instrument according to Claim 16,
**characterised** in that said material is a "shape-memory" material.

18. Instrument according to any of the Claims 1 to 17,
**characterised** in that said jaws (1', 1") are articulated on at least one joint, and that said jaws are operated via a connecting rod or a drawbar.

19. Instrument according to any of the Claims 1 to 18,
**characterised** in that said mounts of said jaws (1', 1") equally serve as feeders for said jaws serving as electrodes and are electrically insulated from the environment.

20. Instrument according to any of the Claims 1 to 19,
**characterised** in that the instrument is provided with means for combined connection to the monopolar and bipolar output terminal of said high-frequency generator.

21. Instrument according to any of the Claims 1 to 20,
**characterised** in that the instrument is a multi-functional instrument for bipolar severing, monopolar or bipolar coagulation, for seizing, rinsing or irrigating, and possibly for cutting by means of the blade of said slide.

22. Instrument according to Claim 21,
**characterised** in that the instrument comprises a multi-functional handle known per se, which is suitable for controlling the individual functions.

## Revendications

1. Instrument chirurgical à haute fréquence comprenant deux électrodes (1', 1") configurées sous forme de mâchoires d'une pince, auquel un courant HF d'un générateur HF est applicable d'une façon qu'un courant haute-fréquence s'écoule entre les mâchoires à travers la région de tissu à traiter, et qu'au moins un canal soit formé, qui fait fonction d'un canal de lavage/d'irrigation et/ou de canal de sortie,
**caractérisé** en ce que pour le coupage et la coagulation à volonté une entretoise (5) électriquement non-conductrice est disposée, qui est introduite entre lesdites électrodes quand le tissu est détaché, de façon que les deux mâchoires de pince soient écartées l'une de l'autre par un écart minimal au cours de l'opération de coupage.

2. Instrument selon la revendication 1,
**caractérisé** en ce que lesdites mâchoires (1', 1") de ladite pince, qui font fonction des électrodes, se terminent dans une pointe.

3. Instrument selon la revendication 2,
**caractérisé** en ce que lesdites mâchoires (1', 1") ont une largeur en amont de la pointe, qui est plus petite que la largeur dans leur zone arrière.

4. Instrument selon la revendication 2 ou 3,
**caractérisé** en ce que ladite pointe renferme un angle relatif à l'axe longitudinal de l'instrument.

5. Instrument selon la revendication 4,
**caractérisé** en ce que lesdites mâchoires (1', 1") sont échangeables de façon qu'on puisse introduire des mâchoires aux angles de pointe différents.

6. Instrument selon la revendication 4 ou 5,
**caractérisé** en ce que ledit angle correspond à 0°, 30°, 90°, 120° or 180°.

7. Instrument selon la revendication 6,
**caractérisé** en ce qu'au cas d'une pointe coudée par 180°, la pointe se trouve au-dedans du contour des mâchoires.

8. Instrument selon une quelconque des revendications 1 à 7,
**caractérisé** en ce que ladite entretoise (5) est un coulisseau.

9. Instrument selon la revendication 8,
**caractérisé** en ce que ladite entretoise (5) présente une lame.

10. Instrument selon une quelconque des revendications 1 to 9,
**caractérisé** en ce qu'au cours d'une opération de coupage, l'écart entre lesdites électrodes (1', 1") corresponde à 1 mm environ.

11. Instrument selon une quelconque des revendications 1 à 10,
**caractérisé** en ce que lesdits supports de fixation desdites mâchoires (1', 1") renferment au moins un canal qui fait fonction d'un canal de lavage/d'irrigation et/ou d'un canal de sortie.

12. Instrument selon une quelconque des revendications 1 à 11,
**caractérisé** en ce que lesdites mâchoires (1', 1") de la pince sont insérées dans un conduit extérieur.

13. Instrument selon la revendication 12,
**caractérisé** en ce que ledit canal, qui fait fonction d'un canal de lavage/d'irrigation et/ou d'un canal de sortie, est formé dans ledit conduit extérieur.

14. Instrument selon la revendication 12 ou 13,
**caractérisé** en ce que lesdites mâchoires (1', 1") consistent en un matériau élastique recourbé et sont fermées par un déplacement desdites mâchoires relativement audit conduit extérieur.

15. Instrument selon la revendication 14,
**caractérisé** en ce que ledit conduit extérieur est déplacé en sens avant afin de fermer lesdites mâchoires (1', 1").

16. Instrument selon la revendication 14 or 15,
**caractérisé** en ce que lesdites mâchoires (1', 1") consistent en un matériau super-élastique.

17. Instrument selon la revendication 16,
**caractérisé** en ce que ledit matériau est un matériau "à mémoire de forme.

18. Instrument selon une quelconque des revendications 1 à 17,
**caractérisé** en ce que lesdites mâchoires (1', 1") sont articulées sur au moins un joint, et en ce que lesdites mâchoires sont actionnées moyennant une bielle de poussée ou un timon.

19. Instrument selon une quelconque des revendications 1 à 18,
**caractérisé** en ce que lesdits supports de fixation desdites mâchoires (1', 1") font également fonction de lignes d'alimentation pour lesdites mâchoires servant en tant que des électrodes, et sont électriquement isolés de leur environnement.

20. Instrument selon une quelconque des revendications 1 à 19,
**caractérisé** en ce que l'instrument est équipé des moyens de connexion combinée aux bornes de sortie mono polaire et bipolaire dudit générateur haute-fréquence.

21. Instrument selon une quelconque des revendications 1 à 20,
**caractérisé** en ce que l'instrument est un instrument à fonctions multiples pour le coupage bipolaire, la coagulation mono polaire ou bipolaire, pour la saisie, le lavage ou l'irrigation, et éventuellement à couper moyennant la lame dudit coulisseau.

22. Instrument selon la revendication 21,
**caractérisé** en ce que l'instrument comprend une poignée à fonctions multiples, connue en soi, qui est apte à commander les fonctions individuelles.
